# EUROPEAN PATENT APPLICATION

(11) **EP 2 251 413 A1**
(43) Date of publication of application: **17.11.2010**
(21) Application number: 09717829.7
(22) Date of filing: 02.03.2009
(51) Int. Cl.: C12N 1/19, A23L 1/221, A23L 1/28, C12P 1/02, C12N 15/09

(54) **GAMMA-GLUTAMYLCYSTEINE-PRODUCING YEAST, AND METHOD FOR PRODUCTION OF YEAST EXTRACT**

(30) Priority: 04.03.2008 JP 2008053424
(71) Applicant: Ajinomoto Co., Inc., Tokyo 104-8315 (JP)
(72) Inventor: NISHIUCHI, Hiroaki, Kawasaki-shi Kanagawa 210-8681 (JP); SUEHIRO, Mariko, Kawasaki-shi Kanagawa 210-8681 (JP)
(74) Representative: Strehl, Peter
(86) International application number: PCT/JP2009/054364
(87) International publication number: WO 2009/110624

(57) **Abstract**

Provided are a yeast capable of efficiently producing γ-glutamylcysteine, a method for producing a yeast with γ-glutamylcysteine accumulated therein, and a method for producing a yeast extract by the use of the yeast. The means for resolution comprises breeding a yeast having a γ-glutamylcysteine-producing ability and having resistance to cerulenin, cultivating the yeast in a medium to accumulate γ-glutamylcysteine in the yeast, and extracting it in hot water to produce a yeast extract.

## Description

### TECHNICAL FIELD

The present invention relates to a yeast capable of producing γ-glutamylcysteine, a method for production of a yeast extract using a yeast having accumulated γ-glutamylcysteine therein, and food and drink containing the yeast extract. γ-Glutamylcysteine is useful in the field of food and drink, drugs and medicines, chemical products, especially in the field of seasonings.

### BACKGROUND ART

At present, sulfur-containing compounds are used broadly in various fields of food products, drugs and medicines, chemical products and the like because of their high potency. For example, glutathione (GSH), a tripeptide comprising glutamic acid, cysteine and glycine is known to have a medicinal potency. At present, a GSH preparation as a medicinal product is positioned as an antidote and an ophthalmic agent, and is used for treatment for various addictions and chronic liver disorders, for prevention of side-effects of anticancer agents and disorders in radiation therapy, and for treatment for skin diseases, cataract and corneal damage (Non-Patent Reference 1).

For food products, glutathione is known as a substance capable of making food products rich in kokumi flavor (Non-Patent Reference 2) ; and a glutathione-rich yeast extract is used in seasonings.

On the other hand, cysteine, a type of a sulfur-containing compound, is used in food products mainly for the purpose of enriching the flavor of food products. For example, there is disclosed a method of preventing food products from browning by dipping food products in an electrolytic solution containing cysteine and its oxidized disulfide, cystine (Patent Reference 1). According to the invention, disclosed is a fact that cystine is reduced into cysteine at the reduction electrode, and the cysteine inhibits the formation of brown pigment via purification of quinones through enzymatic oxidation.

Also disclosed is a technique of using a cysteine residue obtained by reducing an oxidized cysteine residue derived from an animal/plant protein, for improving bread dough (Patent Reference 2). Further, in Examples 1 and 2 in a patent reference that discloses a method for producing a meat-like flavor, it is said that a meat flavor was enriched by mixing chicken or port and cysteine or the like and heating them (Patent Reference 3).

As in the above, for producing cysteine useful in the field of seasonings, known are protein degradation methods and semisynthetic methods, and these methods are mainly used at present. However, with the recent increase in consumers' natural orientation, there is increasing a great demand for cysteine-rich food materials for use of cysteine in food products, but such cysteine-rich food materials have heretofore been unknown.

The present inventors have specifically noted a yeast rich in γ-glutamylcysteine, a dipeptide comprising glutamic acid and cysteine, and have reported production of a cysteine-rich food material by heat treatment or enzymatic treatment of the yeast extract under a specific condition (Patent Reference 4).

In addition, there is reported production of a good flavor composition by adding a saccharide to a sulfur-containing compound, γ-glutamylcysteine followed by heat-treating it (Patent Reference 5). Further, there is reported production of a roast meat flavor-like seasoning by adding a saccharide to a yeast extract containing from 2 to 20% by weight of a sulfur-containing compound such as glutathione, cysteine, glutamylcysteine or the like, followed by heat-treating it in the absence of a fatty acid (Patent Reference 6).

Regarding γ-glutamylcysteine that can be utilized in such methods, some investigations have heretofore been made, for which, it is considered that the following two technical improvements would be needed.

One is to increase the γ-glutamylcysteine content in a yeast cell body to thereby increase the titer therein. Another relates to a cysteine-containing material prepared from a yeast containing γ-glutamylcysteine. The prepared material is rich in cysteine and is usable for enriching the flavor of food products, but owing to the characteristics of the preparation process thereof, the material has the taste and flavor peculiar to the yeast in addition to that of cysteine. Accordingly, in case where a large quantity of the material is added to food products, the food products shall be given the yeast flavor in addition to the potency of cysteine; and therefore, as compared with a case where pure cysteine is added to food products, there still remains a problem to be solved in that the amount of the material to be added is limited. In that background, a technical improvement is desired for more reducing the taste and flavor peculiar to the yeast.

However, the usefulness of γ-glutamylcysteine has just become clarified recently, as compared with that of glutathione, and a means for efficiently producing it has not as yet been investigated so much. Up to the present, the preceding knowledge is as follows:
For example, the present inventors have reported production of a γ-glutamylcysteine-rich yeast by disrupting or attenuating a glutathione synthetase that biosynthesizes glutathione with γ-glutamylcysteine and glycine serving as the substrate (Patent Reference 3, Patent References 7 to 10). According to these techniques, it is intended to retard the metabolism from γ-glutamylcysteine to glutathione and to make the yeast rich in the precursor, γ-glutamylcysteine. However, the γ-glutamylcysteine content is from 1 to 2% by weight, and there still remains room for further improvement in efficiency.

The present inventors have also disclosed a technique of increasing the γ-glutamylcysteine level in a yeast cell body by cultivating a yeast having the ability of producing γ-glutamylcysteine and requiring pantothenic acid, in a pantothenic acid-containing culture medium, and then cultivating it in pantothenic acid-free culture medium (Patent Reference 11). This reference discloses a yeast containing a little less than 4% of γ-glutamylcysteine, and this may be considered as a means for efficiently producing γ-glutamylcysteine. However, for making the yeast stably contain a high level of γ-glutamylcysteine, the amount of pantothenic acid during the cultivation must be controlled.

Further, the present inventors have reported that the γ-glutamylcysteine accumulation level and the yeast growing speed can be both satisfied by making the yeast keep a MET30 genetic product in which the 569th serine residue of the protein as encoded by the gene is mutated into a different amino acid residue (Patent Reference 12).

On the other hand, studies about cerulenin resistance of yeast have been made, and it has been reported that at least a YAP1 gene (also known as an alias, PDR4 gene) (Non-Patent Reference 3) and a FAS2 gene (Non-Patent Reference 4) are concerned in cerulenin resistance. It has been reported that the YAP1 gene is a transcription factor for a GSH1 gene (γ-glutamylcysteine synthetase gene) and a GSH2 gene (glutathione synthetase gene) (Non-Patent Reference 5); but it is not as yet confirmed how the GSH or γ-glutamylcysteine content in an yeast cell body would change through modification of the YAP1 gene therein. The FAS2 gene codes for an α-subunit in fatty acid synthesis, and forms a 6-mer with a β-subunit encoded by the FAS1 gene (Non-Patent Reference 6). Cerulenin resistance could be imparted to a yeast through genetic mutation of such a fatty acid synthetase gene, FAS2 gene; and therefore it could not always be said that cerulenin resistance would change the γ-glutamylcysteine content. Further, there is a report of using a cerulenin-resistant yeast in refined sake production (Patent Reference 13), but there is no report indicating impartation of cerulenin resistance to a yeast having an ability of producing γ-glutamylcysteine, especially to a yeast of which the glutathione synthetase activity is lowered or lost.

In addition, there is also no report indicating what type of taste and flavor the yeast extract derived from such a cerulenin-resistant yeast would have.
Patent Reference 1
JP-A 10-136883
Patent Reference 2
WO 93/08274
Patent Reference 3
EP 0136428
Patent Reference 4
WO 00/30474
Patent Reference 5
Japanese Patent 2830415
Patent Reference 6
Japanese Patent 2903659
Patent Reference 7
WO 01/090310
Patent Reference 8
JP-A 2003-159048 Patent Reference 9
JP-A 2003-159049
Patent Reference 10
WO 2003/080832
Patent Reference 11
JP-A 2004-201677
Patent Reference 12
JP-A 2004-113155
Patent Reference 13
JP-A 8-23954
Non-Patent Reference 1 Protein Nucleic Acid Enzyme 1988-7 VOL. 33, NO. 9, ISSN 003909450 Extra Edition, "Epoch of Glutathione Studies", p. 1626
Non-Patent Reference 2
Y. Ueda et al., Biosci. Biotech. Biochem., 61, 1977-1980 (1997)
Non-Patent Reference 3
Nobusige Nagazawa et al., Journal of Fermentation and Bioengineering Vol. 76, No. 1, 60-63, 1993
Non-Patent Reference 4
Kazuo Aritomi et al., Biosci. Biotechnol. Bochem., 68(1), 206-214, 2004
Non-Patent Reference 5
Sugiyama et al., Journal of Biological Chemistry (2000), 275(20), 15535-15540
Non-Patent Reference 6
Schweizer, E. In Ratledge, C. and Wilkinson, S. G. (eds), Microbial Lipids. Academic Press, London and New York, Vol. 2, pp. 3-50 (1989)

### DISCLOSURE OF THE INVENTION

The problems that the invention is to solve are to provide a yeast capable of efficiently producing γ-glutamylcysteine, to provide a method for production of a yeast extract using the yeast having γ-glutamylcysteine accumulated therein, and to provide food and drink containing the yeast extract and having good flavor and taste.

The present inventors have assiduously studied and, as a result, have found that a yeast having the ability of producing γ-glutamylcysteine and having resistance to cerulenin can stably and efficiently produce γ-glutamylcysteine, and have completed the present invention.

Specifically, the invention is as follows:
(1) A yeast having the ability of producing γ-glutamylcysteine and having resistance to cerulenin.
(2) The yeast of (1), having resistance to at least 10 µM cerulenin.
(3) The yeast of (2), having come to have resistance to at least 10 µM cerulenin as having a mutant FAS2 gene.
(4) The yeast of (3), wherein the mutant FAS2 gene is such that the 1475th amino acid in the protein encoded by the natural FAS2 gene of SEQ ID NO: 5 in the Sequence Listing is lysine and/or the 1800th amino acid therein is asparagine.
(5) The yeast of any one of (1) to (4), which gained the γ-glutamylcysteine producing ability through modification thereof to lower or lose the intercellular glutathione synthetase activity.
(6) The yeast of any one of (1) to (5), which belongs to the genus *Saccharomyces.*
(7) The yeast of (6) wherein the yeast is *Saccharomyces cerevisiae.*
(8) A method for producing a yeast with γ-glutamylcysteine accumulated therein, by cultivating the yeast of any one of (1) to (7) in a culture medium.
(9) A method for producing a yeast extract by cultivating the yeast of any one of (1) to (7) in a culture medium to thereby accumulate γ-glutamylcysteine in the yeast, and then extracting the yeast.
(10) The method for producing a yeast extract of (9), wherein γ-glutamylcysteine is accumulated in the yeast, then the yeast cells are washed at a low temperature, and thereafter the yeast is extracted to give the yeast extract.
(11) A method for producing a yeast extract, comprising further processing the yeast extract of (9) or (10) for heat treatment or enzymatic treatment to thereby change a part or all of γ-glutamylcysteine in the yeast extract into cysteine.
(12) The method for producing a yeast extract of (11), wherein the cysteine content per the yeast extract on the dry matter base is at least 3.5% and the basic amino acid content is at most 1.0%.
(13) An food or drink produced by mixing the yeast extract of any one of (9) to (12) in a raw material of food or drink, and then processing it.
(14) The method for producing a yeast extract of (9), wherein the step of accumulating γ-glutamylcysteine in a yeast, then washing the yeast cells at a low temperature and extracting them to give an yeast extract includes a step of heating the yeast cells in two stages each having a different temperature range to give the extract.
(15) The method for producing a yeast extract of (14), wherein in the step of heating the yeast cells in two stages each having a different temperature range to give the extract, the first-stage extraction temperature is from 25 to 60°C and the second-stage extraction temperature is from 65 to 99°C.

The yeast of the invention can efficiently produce γ-glutamylcysteine. In particular, in a preferred embodiment, when the yeast cells are cultivated at a high density, the increase with time in the γ-glutamylcysteine accumulation amount therein is higher than that in a yeast not having resistance to cerulenin. Further, the yeast extract derived from the yeast of the invention is suitable for a food flavor enriching material, as having little taste or flavor peculiar to the yeast.

### BRIEF DESCRIPTION OF THE DRAWINGS

Fig. 1 is a graph showing the relationship between the cultivation time of yeast and the γ-glutamylcysteine content thereof.
Fig. 2 is a graph showing the relationship between the cultivation time of yeast and the γ-glutamylcysteine content thereof.
Fig. 3 is a graph showing the supernatant Brix change during washing of yeast cells.
Fig. 4 is a view showing a test method for measuring the dissolved gas amount in a yeast cell broth.
Fig. 5 is a graph showing the extraction ratio in two-stage extraction of AJ14800 strain (cerulenin-nonresistant strain).
Fig. 6 is a graph showing the extraction ratio in two-stage extraction of AJ14892 strain (cerulenin-resistant strain).

### BEST MODE FOR CARRYING OUT THE INVENTION

### <1> Yeast of the Invention:

The yeast of the invention has the ability of producing γ-glutamylcysteine and has resistance to cerulenin. The yeast of the invention can be obtained by making a yeast having the ability of producing γ-glutamylcysteine resistant to cerulenin. The yeast of the invention can also be obtained by making a cerulenin-resistant yeast have the ability of producing γ-glutamylcysteine, or by enhancing the γ-glutamylcysteine-producing ability of a cerulenin-resistant yeast. The yeast of the invention can also be obtained by mutating the FAS2 gene of a yeast having the ability of producing γ-glutamylcysteine.

In the invention, "having the ability of producing γ-glutamylcysteine" means that an yeast has the ability of producing γ-glutamylcysteine and accumulating it in the cell body thereof, preferably, accumulating a larger amount of γ-glutamylcysteine therein than a wild yeast strain. More preferably, when the yeast of the invention is cultivated in a minimal medium, it accumulates γ-glutamylcysteine therein in an amount of at least 1% by weight per the dry yeast cell body in its logarithmic growth phase, more preferably at least 1.4% by weight. Also preferably, the glutathione accumulation in the yeast is at most 0.1% by weight per the dry yeast cell body.

The yeast of the invention is not specifically defined so far as it has the ability of producing γ-glutamylcysteine. For example, there may be mentioned the genus *Saccharomyces* such as *Saccharomyces cerevisiae,* etc.; the genus *Candida* such as *Candida utilis,* etc.; the genus *Pichia* such as *Pichia pastoris,* etc.; the genus *Schizosaccharomyces* such as *Schizosaccharomyces pombe,* etc. Of the above yeasts, preferred are those for which the technique of enhancing the γ-glutamylcysteine-producing ability thereof, for example, a method of disrupting or attenuating a glutathione synthetase has been known and for which the γ-glutamylcysteine synthetase gene has been cloned, for constructing the yeast of the invention. As the yeast of the type, preferred are *Saccharomyces cerevisiae* and *Candida utilis.*

The yeast having the ability of producing γ-glutamylcysteine includes, for example, a yeast of which the intercellular glutathione synthetase activity has been lowered or lost, or a yeast so modified as to have an enhanced γ-glutamylcysteine synthetase activity, or a yeast of which the intercellular glutathione synthetase activity has been lowered or lost and which has been so modified as to have an enhanced γ-glutamylcysteine synthetase activity.

The yeast of which the intercellular glutathione synthetase activity has been lowered or lost can be created, for example, by gene replacement using a DNA that contains a gene modified so as to have lost the partial sequence of a gene coding for a glutathione synthetase (GSH2) and so as not to produce the normally-functioning enzyme, or contains a gene having mutation of lowering the enzymatic activity (hereinafter this is simply referred to as "mutant GSH2 gene"). The yeast of which the intercellular glutathione synthetase activity has been lowered or lost can be obtained by processing a wild yeast for ordinary mutation, for example, through UV irradiation or treatment with a mutation agent such as N-methyl-N-nitrosoguanidine (NTG), ethyl methanesulfonate (EMS), nitrous acid, acridine or the like, as in Examples. The resulting mutants having the intended mutation can be confirmed, for example, through PCR or the like.

The above-mentioned mutation to lower the glutathione synthetase activity includes, for example, mutation of replacing the 370th arginine residue with a termination codon (see SEQ ID NO: 1 and 2 in Sequence Listing).

The other mutations to lower the glutathione synthetase activity include the following mutations (see WO03/046155).
(1) Mutation to replace the 47th threonine residue with an isoleucine residue (see SEQ ID NO: 1 and 2 in Sequence Listing).
(2) Mutation to replace the 387th glycine residue with an aspartic acid residue (see SEQ ID NO: 1 and 2 in Sequence Listing).
(3) Mutation to replace the 54th proline residue with a leucine residue (see SEQ ID NO: 1 and 2 in Sequence Listing).

The above mutations may be employed singly or in any desired combination so far as the mutation includes at least (1) or (2). Preferred are a combination of (1) and (3), and a combination of (2) and (3).

Introduction of the intended mutation into a glutathione synthetase gene may be attained according to a site-specific mutation method using a synthetic oligonucleotide.

The gene replacement may be attained as follows: A yeast is transformed with a recombinant DNA containing a mutant GSH2 gene, thereby inducing recombination between the mutant GSH2 gene and the GSH2 gene on the chromosome. In this stage, the recombinant DNA may be made to contain a marker gene in accordance with the character such as auxotrophy or the like of the host for facilitating the operation. The recombinant DNA may be cleaved with a restriction endonuclease or the like to be linear, and further, the replication control region that functions in the yeast may be removed, whereby the strain with the recombinant DNA inserted in the chromosome therein can be efficiently isolated.

For the genetic transformation of the yeast, employable is an ordinary method for yeast transformation, such as a protoplast method, a KU method, a KUR method, an electroporation method or the like.

The strain with the recombinant DNA inserted into the chromosome therein as above undergoes recombination between the mutant GSH2 gene and the GSH2 gene originally existing in the chromosome, and in this, two fused genes of the wild GSH2 gene and the mutant GSH2 gene are inserted in the chromosome in such a manner that they sandwich the other parts (vector part an marker gene) of the recombinant DNA therebetween.

For the purpose of leaving the mutant GSH2 gene alone of the two fused genes, one copy of the GSH2 gene is removed from chromosomal DNA along with the vector part (including the marker gene) through recombination of the two GSH2 genes. This stage includes a case where the wild GSH2 gene is left on the chromosomal DNA and the mutant GSH2 gene is cut out, and a case where on the contrary, the mutant GSH2 gene is left on the chromosomal DNA and the wild GSH2 gene is cut out. In any case, the marker gene is removed, and therefore the second stage recombination can be confirmed by the phenotypic character corresponding to the marker gene. In the intended gene-replaced strain, the GSH2 gene can be amplified through PCR and its structure can be identified whereby the strain can be selected.

The mutant GSH2 gene for use in the genetic replacement may code for a full-length glutathion synthetase protein but may code for a part of the protein so far as it includes the deleted site.

The base sequence of the glutathione synthetase gene (GSH2) of *Saccharomyces cerevisiae* has been reported (Inoue et al., Biochim. Biophys. Acta, 1395 (1998), 315-320, GenBank Accession Y13804, SEQ ID NO: 1), and through PCR using the oligonucleotide based on that base sequence as a primer, the gene can be isolated from the chromosomal DNA of *Saccharomyces cerevisiae.* The gene to be introduced may be a gene derived from any other microorganisms than the genus *Saccharomyces.*

The mutant GSH2 gene for use in the invention may code for a GSH2 product that has an amino acid sequence including replacement, deletion, insertion or addition of one or more amino acids in one or a few sites, in addition to the above-mentioned 47-position, 387-position and 54-position mutation, in the amino acid sequence shown by SEQ ID NO: 2 in the Sequence Listing. The wording "a few" as referred to herein may differ depending on the position and the type of the amino acid residue in the steric configuration of protein, but concretely, for example, the wording "a few" may be from 2 to 10, preferably from 2 to 6, more preferably 2 to 3. The mutant GSH2 gene may be a DNA that codes for a protein having homology of at least 70%, more preferably at least 80%, even more preferably at least 90%, still more preferably at least 95% to the entire amino acid sequence of the GSH2 product.

The above-mentioned base substitution, deletion, insertion, addition, inversion and the like include naturally-occurring mutation (mutants or variants), for example, based on the individual difference as well as the difference in the species and the genus of the microorganisms that hold the GSH2 gene.

In case where the GSH2 gene is disrupted, the mutant GSH2 gene for use for genetic replacement may not always contain the full length thereof, but may has the length necessary for genetic disruption. Not specifically defined, the microorganisms for use for obtaining the GSH2 gene may have the homology to such a degree that the gene could induce homologous recombination with the homologous gene of the microorganism used in creation of the gene-disrupted strain.

As the DNA capable of inducing homologous recombination with the GSH2 gene of *Saccharomyces cerevisiae,* concretely there is mentioned a DNA that has homology of at least 70%, preferably at least 80%, more preferably at least 90%, even more preferably at least 95% to the DNA having the sequence shown in SEQ ID NO: 1. More concretely, there is mentioned a DNA capable of hybridizing with the DNA having the base sequence shown in SEQ ID NO: 1 under a stringent condition. The stringent condition includes a condition for washing at 60°C and at a salt concentration corresponding to 1 x SSC, 0.1% SDS, preferably 0.1 x SSC, 0.1% SDS.

The yeast of which the glutathione synthetase activity has been lowered or lost can be obtained by processing a wild yeast for ordinary mutation, for example, through UV irradiation or treatment with a mutation agent such as N-methyl-N-nitrosoguanidine (NTG), ethyl methanesulfonate (EMS), nitrous acid, acridine or the like, as in Examples.

The method of enhancing the intracellular γ-glutamylcysteine synthetase activity in the yeast includes transforming the yeast with a plasmid having the enzyme gene (GSH1) (for example, γ-glutamylcysteine synthetase gene of *Saccharomyces cerevisiae:* GenBank Accession No. D90220) inserted therein to thereby increase the number of copies in the cell of the gene, or replacing the promoter of the γ-glutamylcysteine synthetase gene on the chromosome with a strong transcriptional promoter (Yasuyuki Ohtake, et al., Bioscience and Industry, Vol. 50, No. 10, pp. 989-994, 1992). As the γ-glutamylcysteine synthetase gene, known is a gene that codes for the glutathione synthetase of Candida utilis (JP-A 2005-073638), and this can be used in the present invention.

The intercellular γ-glutamylcysteine synthetase activity and glutathione synthetase activity can be measured according to a Jackson's method (Jackson, R. C., Biochem. J., 111, 309 (1969)) or a Gushima et al's method (Gushima, T. et al., J. Appl. Biochem., 5, 210 (1883)), respectively.

By impartation of MNNG resistance (JP-A 2003-159048), retention of mutant MET30 gene (JP-A 2004-113155), desuppresion of MET25 gene expression (JP-A 2004-201677), impartation of pantothenic acid requirement (JP-A 2004-201677), the γ-glutamylcysteine-producing ability of the yeast can be enhanced, or the yeast can have desired properties.

The yeast of the invention is a yeast strain which has been so modified that it has the above-mentioned ability of producing γ-glutamylcysteine and has cerulenin resistance. The wording "having cerulenin resistance" as referred to in the invention means that the yeast strain can grow in a medium that contains cerulenin to a degree of concentration in which the non-modified yeast strain, for example, the wild strain or the parent strain used in obtaining the yeast of the invention could not grow. Concretely, for example, in case where a suspended yeast culture is spread on an agar medium such as a YPD plate or the like containing cerulenin, and cultivated therein at a favorable temperature, for example, at 30°C, and when the non-modified strain does not form a colony under the condition but the modified strain forms a colony, then the modified strain has cerulenin resistance. "Colony" as referred to herein means a colony that can be confirmed with the naked eye.

| (YPD Medium Composition) | |
|---|---|
| Glucose | 2% |
| Peptone | 2% |
| Yeast extract | 1% |
| (pH 5.0) | |
| (In an agar medium, the agar content is 2%). | |

A case of *Saccharomyces cerevisiae* as the yeast is described more concretely. The yeast is inoculated in the YPD medium, and cultivated at the growth-optimal temperature, for example at 30°C for 3 days; the resulting culture is spread on a YPD plate containing a certain concentration of cerulenin, and cultivated thereon at the growth-optimal temperature. Under the condition, when the yeast forms a colony within 5 days, then the yeast has resistance to cerulenin at that concentration. Cells of *Saccharomyces cerevisiae* not modified to have resistance to cerulenin, for example, those of the AJ14800 strain described in Examples form few colonies on a YPD plate containing 7 µM cerulenin, within 3 days. Preferably, the yeast of the invention has resistance to at least 10 µM, more preferably at least 15 µM, even more preferably at least 20 µM cerulenin.

The yeast having resistance to cerulenin can be obtained, for example, by mutating a wild strain of the yeast or a yeast having the ability of producing γ-glutamylcysteine, then inoculating the resulting mutant in an agar medium containing cerulenin to a degree of concentration in which the wild strain could not grow, and selecting the strain having formed a colony. The culture of the thus-selected strain is again spread on an agar medium containing cerulenin, and the strain having the ability of forming a colony is selected; and this process may be repeated twice or more. Further, the thus-obtained cerulenin-resistant yeast may be again mutated and processed according to the operation mentioned above. The mutation may be ordinary mutation like that for the yeast of which the glutathione synthetase activity has lowered or lost, for example, UV irradiation or treatment with a mutation agent such as N-methyl-N-nitrosoguanidine (NTG), ethyl methanesulfonate (EMS), nitrous acid, acridine or the like.

The cerulenin-resistant yeast may also be obtained as a natural mutant from a wild strain not processed for mutation, by cultivating the wild strain in a cerulenin-containing medium in the same manner as above.

The yeast of the invention includes a yeast of the above-mentioned cerulenin-resistant yeast, which has gotten a mutant FAS2 gene and therefore has come to be resistant to cerulenin. The wording "getting a mutant FAS2 gene" means introduction of mutation so as to impart cerulenin resistance to the amino acid sequence of the protein encoded by a wild FAS2 gene. As one example, it is known that, in the amino acid sequence of SEQ ID NO: 5 and 6 of Sequence Listing that *Saccharomyces cerevisiae* has, cerulenin is bound to the sequence site of TPVGAC from the 1300th amino acid to the 1305th amino acid (Hiroshi Funabashi, et al., J. Biochem., 105, 751-755 (1989)), to thereby retard the activity of the protein encoded by the FAS2 gene. It is considered that, since cerulenin is directly bound to the amino acid sequence, especially to the 1305th cysteine residue, the mutation at that site would make the resulting yeast have resistance to cerulenin. It is further known that the protein encoded by the FAS2 gene that *Candida albicans* has also has the amino acid sequence of TPVGAC (Susan B. Southard, et al., Gene, 156 (1995), 133-138). In addition, there is further mentioned as other examples, mutation of changing the 1250th glycine into serine, alanine or cysteine in the amino acid sequence of SEQ ID NO: 5 and 6 (Kazuo ARITOMI, et al., Biosci. Biotechnol. Biochem., 68(1), 206-214 (2004)). Further mentioned as still other examples are mutation of changing the 1475th glutamic acid into lysine, and mutation of changing the 1800th serine into asparagine.

In the manner as above, the mutant FAS2 gene is not specifically defined so far as its mutation is for adding cerulenin resistance to the amino acid sequence of the protein encoded by the wild FAS2 gene. Especially preferred is the mutation of changing the 1250th glycine, the 1305th cysteine, the 1417th serine, the 1475th glutamic acid or the 1800th serine in the amino acid sequence of SEQ ID NO: 5 in the Sequence Listing, into any other amino acid.

For example, preferred are mutation of the 1250th glycine into serine, alanine or cysteine, mutation of the 1475th glutamic acid into lysine, and mutation of the 1800th serine into asparagine. More preferred is introduction of mutation of the 1475th glutamic acid into lysine and/or mutation of the 1800th serine into asparagine. Further, most preferred is introduction of both mutation of the 1475th glutamic acid into lysine and mutation of the 1800th serine into asparagine.

The mutant FAS2 gene in the invention may have an amino acid sequence including substitution, deletion, insertion or addition of one or a few amino acids in one or a few sites in the amino acid sequence of SEQ ID NO: 5. The wording "a few" as referred to herein may differ depending on the position and the type of the amino acid residue in the steric configuration of protein, but concretely, for example, the wording "a few" may be from 2 to 20, preferably from 2 to 10, more preferably 2 to 5.

The mutant FAS2 gene may coexist with an ordinary wild gene so far as it contributes toward cerulenin resistance.

The yeast of the invention may be a monoploid but preferably has diploidic or more polyploidy as its growth is good. The yeast having diploidic or more polyploidy may be obtained by mutating a yeast having polyploidy followed by selecting a strain capable of producing γ-glutamylcysteine, or by mating a monoploidic yeast used in breeding a γ-glutamylcysteine-producing strain with a monoploid of a wild yeast, then sporulating the resulting diploidic yeast, selecting a strain that produces γ-glutamylcysteine, and mating the two, γ-glutamylcysteine-producing monoploidic strains each having a different mating type. Similarly, triploidic or more polyploidic yeasts can be obtained.

The operation regarding the yeast breeding and modification as above is described in "Chemistry and Biology Experiment Line 31, Experimental Technique for Yeast" 1st Ed., Hirokawa Shoten; "Bio Manual Series 10 Gene Testing Method with Yeast" 1st Ed., Yodosha; "METHODS in YEAST GENETICS 2000 Edition" Cold Spring Harbor Laboratory Press, etc.

As described above, the cerulenin-resistant yeast efficiently produces γ-glutamylcysteine. Accordingly, based on the cerulenin resistance as the phenotype, a strain capable of efficiently producing γ-glutamylcysteine can be screened or bred.

### <2> Utilization of Yeast of the Invention:

Cultivating the yeast of the invention in a medium provides a yeast with γ-glutamylcysteine accumulated therein, and its culture, and further the yeast extract.

The medium to be used for cultivation of the yeast of the invention is not specifically limited so far as the yeast of the invention can well grow therein and can efficiently produce γ-glutamylcysteine, and in general, a medium for industrial use may be used. If desired, necessary nutrients may be added to the medium in accordance with the character of the yeast used.

For example, regarding a bakery yeast, the culture in which it is cultivated to a logarithmic growth phase or a stationary phase is inoculated into a nutrient medium to be 2% therein, and this is cultivated by shaking therein at 25°C to 35°C for 8 to 24 hours to give a yeast strain in a logarithmic growth phase.

When the culture has reached the intended γ-glutamylcysteine accumulation level, the cultivation is stopped. In general, under a favorable condition, the cultivation time may be from 10 to 30 hours, preferably from 15 to 27 hours.

In the invention, high-density cultivation may be employed as one mode of cultivation. In the invention, "high-density cultivation" means that the cell density in liquid cultivation is at least 1 x 10⁸ cells/ml, preferably at least 2.5 x 10⁸ cells/ml, more preferably at least 4 x 10⁸ cells/ml. Otherwise high-density cultivation includes another case where the dry cell weight in liquid cultivation is at least 1 g/100 ml, preferably at least 2.5 g/100 ml, more preferably at least 4 g/100 ml. The whole period of cultivation may not be high-density cultivation, and the culture may reach the above-mentioned cell density in at least a part of the period. For example, preferably, the culture reaches the above-mentioned cell density in at least 1% of the culture period, preferably at least 5%, more preferably at least 10% thereof.

In high-density cultivation, the yeast of the invention may have an increased γ-glutamylcysteine content per cell with time. Some conventional γ-glutamylcysteine yeasts not having cerulenin resistance may have an increased γ-glutamylcysteine content in high-density cultivation; however, with the lapse of the cultivation period of time, the increase in the γ-glutamylcysteine content in those yeasts may tend to be saturated. As opposed to this, the yeast of the invention provides a remarkable increase in the γ-glutamylcysteine content therein in a preferred embodiment, as compared with such conventional yeasts.

The culture and its fractions thus obtained in the manner as above contain γ-glutamylcysteine. The culture may be the culture liquid containing the yeast cells, but may be the yeast cells, the yeast cell fragments or the yeast cell extract (yeast extract). A fraction containing γ-glutamylcysteine may be separated from the yeast cell fragments or the yeast extract.

In case where the yeast cells are separated from the yeast cells-containing culture and washed, they are processed preferably at a low temperature. Low temperature means a temperature lower than the temperature at which the yeast is cultivated. Specifically, when the cultivation temperature is 30°C, the low temperature means lower than 30°C. More preferably, the temperature is not higher than 20°C, even more preferably not higher than 16°C, most preferably not higher than 10°C. However, in a too low temperature zone, the culture liquid may be frozen and the cells could not be separated from it; and needless-to-say, therefore, the temperature at which the culture liquid is not frozen should be the lower limit. In case where water is used for washing the cells, the lower limit of the temperature must be set at a temperature higher than 0°C.

An ordinary method may be employed for separating the yeast cells from the culture liquid. For example, there may be mentioned centrifugation, membrane separation, filter press, decantation, etc. From the viewpoint of the operability, centrifugation is preferred.

The yeast extract and the like may be prepared in the same manner as that for preparation of ordinary yeast extracts. Specifically, the yeast extract may be obtained by extraction or digestion of the yeast cells. For extraction, the yeast cells (as the case may be, the yeast cells-containing culture liquid) are extracted with hot water. The condition for hot water extraction of the yeast cells is generally at 50 to 99°C, preferably at 60 to 95°C, more preferably at 65 to 90°C.

A part or all of γ-glutamylcysteine may be converted into cysteine through heat treatment or enzymatic treatment of the γ-glutamylcysteine-containing yeast extract. The heating condition in this case is not specifically defined. In general, the yeast extract may be heated at 50 to 120°C for 3 to 300 minutes. For enzymatic treatment, the yeast extract may be processed with a γ-glutamylpeptide hydrolase. The γ-glutamylpeptide hydrolase usable herein concretely includes glutaminase,' γ-glutamyltransferase, γ-glutamylcyclotransferase, etc. One or more these enzymes may be used either singly or as combined.

In hot water extraction of the cerulenin-resistant yeast cells, the cells may be heated in two stages each falling within a different temperature region, or that is, they may be heated in a low-temperature region and then in a high-temperature region whereby the extraction ratio to give the intended extract may be increased. The temperature difference between the low-temperature region and the high-temperature region may be at least 5°C. Preferably, the low-temperature region covers from 25 to 60°C, more preferably from 25 to 55°C, even more preferably from 35 to 55°C. Preferably, the high-temperature region covers from 65 to 99°C, more preferably from 65 to 95°C, even more preferably from 70 to 90°C. In the low-temperature region, the cells may be heated for a predetermined period of time, generally for 20 seconds or more, preferably for 2 minutes or more, even more preferably for 20 minutes or more. When the heating time is longer, the effect could be more remarkable; however, in case where the cells are heated for a too long period of time, it is unfavorable since there is a risk of growth of contaminant microorganisms. The heating time may be set in consideration of the above; and as one example, the upper limit of the heating time may be 15 hours. In the high-temperature region, the cells may also be heated for a predetermined period of time; and for example, the cells may be heated for 30 seconds to 20 minutes, preferably for 1 minute to 20 minutes, more preferably for 1 minute to 15 minutes, even more preferably for 2 to 10 minutes.

Of the yeast extract obtained through heat treatment or enzymatic treatment of the γ-glutamylcysteine-containing yeast extract, preferred is one in which the cysteine content per the yeast-derived extract solid fraction is at least 3.5% and the basic amino acid content is at most 1.0%, as it has little taste and flavor peculiar to the yeast. In the invention, the basic amino acid is meant to indicate histidine, arginine and lysine. More preferably, the cysteine content per the yeast-derived extract solid fraction is at least 3.5% and the basic amino acid content is at most 0.8%. Even more preferably, the cysteine content per the yeast-derived extract solid fraction is at least 3.5% and the basic amino acid content is at most 0.6%.

The above-mentioned, γ-glutamylcysteine or cysteine-containing yeast extract, and the culture obtained by cultivating the yeast having high ability of γ-glutamylcysteine production and its fractions can be used in producing food and drink. Specifically, the above-mentioned, γ-glutamylcysteine or cysteine-containing yeast extract, and the culture obtained by cultivating the yeast having high ability of γ-glutamylcysteine production and its fractions may be, either singly or as combined, mixed with materials for food and drink, and then processed by heating or the like. The food and drink include drinking and eating products such as alcohol drinks, bread products, fermented food seasonings, etc. The production of cysteine from γ-glutamylcysteine through heat treatment may be attained during or after production of food and drink.

The above-mentioned food and drink may be produced by mixing the γ-glutamylcysteine or cysteine-containing yeast extract of the invention, and the culture obtained by cultivating the yeast having high ability of γ-glutamylcysteine production and its fractions of the invention, either singly or as combined, with materials for food and drink, and then processing them into food and drink. The food and drink of the invention may be produced using the same starting materials as those for ordinary food and drink and in the same manner as that for ordinary food and drink, except that the above-mentioned, yeast extract, the culture and the fraction are used.

The starting materials, for example, for alcohol drinks include rice, barley, cornstarch, etc.; and those for bread products include wheat, sugar, salt, butter, yeast for fermentation, etc.; and those for fermented food seasonings include soybean, wheat, etc.

### (EXAMPLES)

The invention is described more concretely with reference to the following Examples. However, the invention is not whatsoever limited by the following Examples.

### (Reference Example 1) Molecular Breeding of High-γ-GC Yeast through High Expression of YAP1 Gene:

From a γ-glutamylcysteine-producing yeast of AJ14800 strain, a strain capable of highly expressing a YAP1 gene was bred.

The AJ14800 strain is a diploidic yeast (gsh2/gsh2) produced by mating a strain AJ14809, which was derived from wild *Saccharomyces cerevisiae* and of which the glutathione synthetase activity was attenuated, and a wild strain of *Saccharomyces cerevisiae* AJ14810 (MATa) and selecting the strain, of which the glutathione synthetase activity was attenuated and which produces γ-glutamylcysteine (JP-A 2003-159048, US Publication 2004-0214308A1).

The strain AJ14809 was deposited on October 1, 2001, in the National Institute of Advanced Industrial Science and Technology, International Patent Organism Depositary (305-5466, Center No. 6, 1-1, Higashi 1-chome, Tsukuba-shi, Ibaragi-ken, Japan) as an accession number of FERM P-18546, and on November 1, 2002, this was transferred to the international depositary authority based on the Budapest Treaty and was given an accession number of FERM BP-8228. The strain AJ14810 was deposited on October 1, 2001, in the National Institute of Advanced Industrial Science and Technology, International Patent Organism Depositary (305-5466, Center No. 6, 1-1, Higashi 1-chome, Tsukuba-shi, Ibaragi-ken, Japan) as an accession number of FERM P-18547, and on November 1, 2002, this was transferred to the international depositary authority based on the Budapest Treaty and was given an accession number of FERM BP-8229.

According to an ordinary method, a vector capable of highly expressing a YAP1 gene (YAP1/pAUR123) was produced as follows: The strain AJ14800 was cultivated in a YPD medium, and its chromosomal DNA was collected in the logarithmic growth phase thereof. Using the collected chromosomal DNA as a template, and using Pyrobest DNA polymerase (by Takara Bio), a primer 1 (SEQ ID NO: 3) and a primer 2 (SEQ ID NO: 4), the ORF region of the YAP1 gene was amplified. The primer terminal was so designed that an SmaI site or an XbaI site could be added thereto, and therefore, an SmaI site is added to the N-terminal side of the PCR product and an XbaI site to the C-terminal side thereof. The PCR product was purified; and the purified product and a protein-expressing shuttle vector for yeast, pAUR123 (by Takara Bio) were cleaved with restriction endonucleases SmaI and XbaI, respectively; and the resulting fragments were ligated using a TaKaRa DNA ligation kit ver. 2 (by Takara Bio). *Escherichia coli* JM109 was transformed with the ligated product to give a transformant. The transformant having the intended expression vector was selected, and a large quantity of YAP1/pAUR123 vector was mass-produced from the obtained strain.

The strain AJ14800 was transformed with the YAP1/pAUR123 vector produced as above, and pAUR123. Concretely, the operation is as follows: The strain AJ14800 was cultivated in a YPD medium, and the cells were collected during the logarithmic growth phase thereof. These were washed twice with 1 M sorbitol solution, and the cells were suspended in a solution having the composition mentioned below, and left at 5°C for 1 hour.

| (Composition) | |
|---|---|
| | 0.1 M LiCl |
| | 10 mM DTT |
| | 10 mM Tris-HC1(pH 7.5) |
| | 1 mM EDTA |

Subsequently, the cells were washed twice with 1 M sorbitol solution. The thus-prepared cells were mixed with the PCR product produced as above, and processed for electroporation ("Biomass Manual Series 10 Gene Testing Method with Yeast" 1st Ed., Yodosha). Subsequently, the mixture was inoculated into a YPD medium, and cultivated by shaking at 30°C for 16 hours. The culture was spread on a YPD agar medium containing 0.2 µg/ml of a pAUR123 vector selection marker, Aureobasidin A (by Takara Shuzo, code 9000), and cultivated at 30°C for 3 days. The minimum growth inhibitory concentration of Aureobasidin A for the strain AJ14800 is 0.05 µg/ml. The formed colonies were applied onto a YPD agar medium containing 0.2 µg/ml of Aureobasidin A, and the colonies resistant to Aureobasidin A were selected. From these colonies, the transformants having the intended vector were selected to be a strain AJ14800/YAP1/pAUR123 and a strain AJ14800/pAUR123.

The above two strains were separately implanted in a YPD medium (4 ml, test tube) containing 0.2 µg/ml of Aureobasidin A, and cultivated by shaking at 30°C. The pre-cultures were separately 2% seeded in an SD medium (50 ml, Sakaguchi flask) containing 0.2 µg/ml of Aureobasidin A, and cultivated by shaking at 30°C. In the logarithmic growth phase (in 16 hours after the seeding), the cells were collected, and the γ-glutamylcysteine content therein per the dry cell was measured. The content in the strain AJ14800/YAP1/pAUR123 was 1.7% by weight; and that in the strain AJ14800/pAUR123 was 1.4% by weight.

The above confirmed that the increase in the expression level of the YAP1 gene brought about the increase in the intracellular γ-glutamylcysteine content.

| (SD Medium Composition) | |
|---|---|
| Glucose | 2% |
| Nitrogen Base | 1-time concentration |

(Ten-times concentration Nitrogen Base is one prepared by dissolving a mixture of 1.7 g of Bacto Yeast Nitrogen Base w/o Amino Acids and Ammonium Sulfate (by Difco) and 5 g of ammonium sulfate in 100 ml of sterile water, then controlling its pH to 5.2 or so, and sterilizing it through filtration.)

Cerulenin-containing YPD agar media each having a different cerulenin concentration were prepared, in which the minimum growth inhibitory concentration of cerulenin for the above two strains was determined. Concretely, each strain was cultivated in a YPD liquid medium at 30°C by shaking, and 3 µl of the culture having reached the stationary phase was spotted onto the cerulenin-containing agar medium, and cultivated at 30°C for 3 days. Based on the presence or absence of colonies, the growth of the cells was evaluated. As a result, the minimum growth inhibitory concentration for the strain AJ14800/YAP1/pAUR123 was 9 µM, and that for the strain AJ14800/pAUR123 was 7 µM. This confirmed that the increase in the expression level of the YAP1 gene brought about the increase in the cerulenin resistance.

### (Example 1)

### (Production of Cerulenin-Resistant Strain)

In the same manner as in Reference Example 1, the minimum growth inhibitory concentration of cerulenin for the strain AJ14800 was determined, and was 7 µM.

Next, the strain AJ14800 was inoculated in a YPD medium and cultivated at 30°C for 3 days. The culture was spread on a YPD plate, a YPD plate containing 7 µM cerulenin, and an YPD plate containing 10 µM cerulenin, thereby giving a cerulenin-resistant strain. The expression ratio of the resistant strain was evaluated by comparing the number of the colonies formed on the YPD plate and that on the cerulenin-containing YPD plates. As a result, on the 7 µM plate, one strain of 100 strains was the resistant strain; and on the 10 µM plate, one strain of 2000 strains was the resistant strain.

### (Example 2) Classification of Cerulenin-Resistant Strain:

The cerulenin-resistant strains obtained in Example 1 were checked for the degree of cerulenin resistance thereof (growth inhibitory concentration), and the results are as follows:

| |
|---|
| Degree of cerulenin resistance, from 7 µM to less than 10 µM: |
| 420 strains |
| Degree of cerulenin resistance, 10 µM or more: |
| 613 strains |

The γ-glutamylcysteine content in these strains was measured, and the strains in which the γ-glutamylcysteine content was higher than that in the parent strain and which grew well were selected. In each group, the following strains were selected.

| |
|---|
| Degree of cerulenin resistance, from 7 µM to less than 10 µM: |
| 8 strains |
| Degree of cerulenin resistance, 10 µM or more: |
| 18 strains |

As the best strains from each group, selected was a strain 7-46 (selected from the group having a degree of cerulenin resistance of from 7 µM to less than 10 µM), and a strain 10-29 (selected from the group having a degree of cerulenin resistance of 10 µM or more). These strains were separately cultivated in an SD medium, and the γ-glutamylcysteine content therein in the logarithmic growth phase was measured. As a result, the content in the strain AJ14800 was 1.50%; that in the strain 7-46 was 1.68% (1.1 times relative to the parent strain); and that of the strain 10-29 was 1.80% (1.2 times relative to the parent strain).

The strain AJ14800 and the strain 10-29 were analyzed for the base sequence of the YAP1 gene therein, but no difference was found out between them. From this, it is presumed that the reason for the increase in the γ-glutamylcysteine content in the strain 10-29 would be any other than the mutation of the YAP1 gene. The strain 10-29 could grow even on the plate containing 25 µM cerulenin.

### (Example 3) γ-Glutamylcysteine Content in High-Density Cultivation:

The strain AJ14800, the strain 7-46 and the strain 10-29 were separately cultivated at high density in a mini-jar, and the γ-glutamylcysteine in each strain was measured.

One platinum loop of each strain was inoculated in 50 ml of a YPD medium (in a 500-ml Sakaguchi flask) , and cultivated at 30°C by shaking at 120 rpm for pre-seed cultivation. Next, 5 ml of the pre-seed culture was inoculated in 500 ml of a YPD medium (in a 2-liter baffle-equipped Erlenmeyer flask) and then cultivated at 30°C by shaking at 120 rpm for 48 hours for seed cultivation. 200 ml of the seed culture was inoculated in a main medium, and cultivated under the condition mentioned below, using a mini-jar (liquid amount, 2 liters).

**Table 1**

| Composition of Main Medium | | |
|---|---|---|
| | Composition | Final Concentration (g/dl) |
| Group A | Glucose | 0.50 |
| | KH₂PO₄ | 0.30 |
| Group B | MgSO₄ | 0.17 |
| | (NH₄)₂SO₄ | 1.00 |
| | Yeast Extract(TN%) | 0.23 |
| | Defoaming Agent* | 0.10 |
| Group C | CaCl₂ | 0.07 |
| *: Mitsubishi Chemical's Ryoto Polyglyester 0-50D | | |

The group A, the group B and the group C were separately autoclave-sterilized (120°C, 20 minutes), and then mixed. Yeast extract was so added that the total nitrogen amount could be 0.23 g/dl. The cultivation temperature was 30°C, the aeration amount was 1/1 VVM and the stirring number was 800 rpm during the cultivation. As the sugar feed liquid, used was 46.7% (w/v) glucose liquid; and when the pH of the culture broth reached 6.0, the feeding was started. During the cultivation, the pH was controlled to be pH 6.0 with ammonia gas, after the start of the sugar feeding. After the sugar feeding, the absorbance (OD 660 nm) of the culture was measured at intervals of 3 hours, and glucose was so added to be 0.2 µg-glucose/dry cell weight (DCW) g/hr. As the formula for conversion, used was DCW = 0.22 x OD 660 nm.

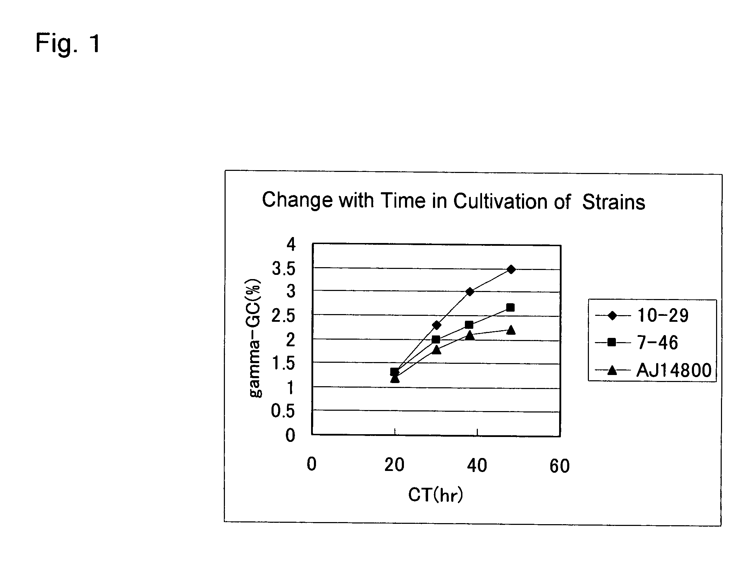

At regular time intervals, the γ-glutamylcysteine content was measured, and the results are shown in Fig. 1. On 48 hours of the cultivation, the dry cell weight (DCW) and the γ-glutamylcysteine content (γ-GC) were measured, and the results are shown below.

| | |
|---|---|
| AJ14800: | γ-GC 2.10%, DCW 3.65 g/dl |
| 7-46: | γ-GC 2.66%, DCW 3.23 g/dl |
| 10-29: | γ-GC 3.48%, DCW 3.16 g/dl |

In the strain 10-29, the γ-glutamylcysteine content noticeably increased with time. In the other strains selected from those having a degree of cerulenin resistance of 10 µM or more, the γ-glutamylcysteine content noticeably increased with time in high-density cultivation.

### (Example 4) Investigation of Further Reduction in Glutathione Synthetase Activity of Strain 10-29:

Next, the glutathione synthetase gene of the strain 10-29 was disrupted to further lower the glutathione synthetase activity thereof, and its influence on the resulting strain was investigated. According to the same method as that described in Example 3 in JP-A 2004-201677, the glutathione synthetase gene of the strain 10-29 was disrupted. The resulting strain was given a private number AJ14892, and this was deposited on August 25, 2006, in the National Institute of Advanced Industrial Science and Technology, International Patent Organism Depositary (305-5466, Center No. 6, 1-1, Higashi 1-chome, Tsukuba-shi, Ibaragi-ken, Japan) as an accession number of FERM P-21007, and on February 19, 2009, this was transferred to the international depositary authority based on the Budapest Treaty and was given an accession number of FERM ABP-11097.

The strain AJ14892 obtained as above was cultivated at high density in the same manner as in Example 3. As the sugar feed liquid, used was 60% glucose solution; and when the pH of the broth reached 6.0, the sugar feeding was started. The sugar feed flow rate was set to be 20 µl/min up to 0.5 hours after the start of the sugar feeding, 75 µl/min up to 5.9 hours, and 135 µl/min after that.

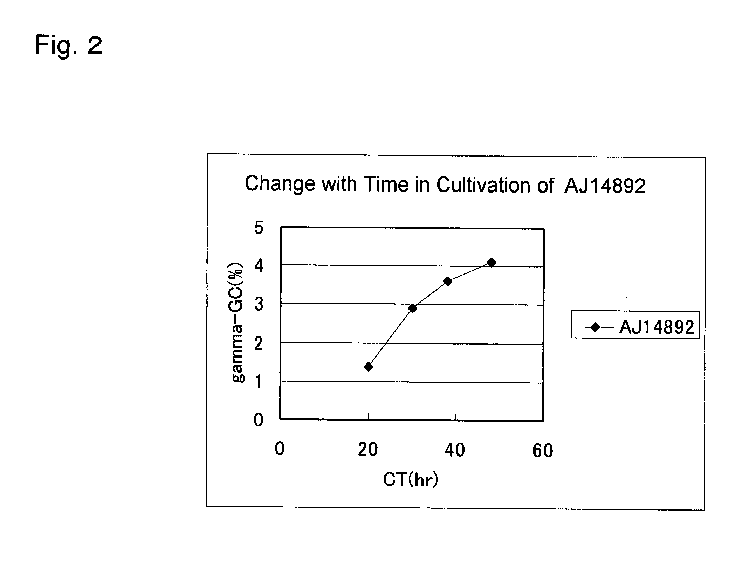

At regular time intervals, the γ-glutamylcysteine content was measured, and the results are shown in Fig. 2. On 48 hours of the cultivation, the dry cell weight (DCW) and the γ-glutamylcysteine content (γ-GC) were measured, and the results are shown below.

| | |
|---|---|
| γ-GC: | 4.12% |
| DCW: | 5.02 g/dl |

Also in this strain like that in the strain 10-29, the γ-glutamylcysteine content noticeably increased with time in the high-density cultivation. From this result, it is known that further reduction in the glutathione synthetase activity also resulted in increase in the γ-glutamylcysteine content with time.

### (Example 5) Preparation of Yeast Extraction composition (yeast extract):

From the culture broth of the strain AJ14800 described in Example 3 and that of the strain AJ14892 described in Example 4, a yeast extraction composition (yeast extract) having a high content of cysteine was prepared according to method mentioned below.

Each culture broth was centrifuged to separate the yeast cells from the medium. After the centrifugation, the yeast cells contained the medium as the mother liquid adhering thereto. Therefore, for the purpose of removing the medium adhering to the cells as the mother liquid, the yeast cells were added to washing water and suspended therein. The resulting suspension was further centrifuged to separate the medium fraction (light liquid) and the yeast cells (heavy liquid). This operation was repeated until Brix of the light liquid reached 0.6. In the laboratory scale, the centrifugation may finish in a short period of time, but in the BP/CP scale with the assumption of an industrial level, the medium removal may take a few hours. Accordingly, in consideration of the BP/CP scale influences thereon, the yeast cells was suspended in a concentration of about 10 g-DCW (dry cell weight)/dl, and with stirring it with a stirrer (at a stirring rate on such a level that the yeast cells would not be settled at the rate), this was kept at 16°C for 4 hours. Subsequently, the yeast cells were collected through centrifugation. The yeast cells were suspended in water to have a concentration of about 12 g-DCW/dl. The yeast cell suspension was heated at 70°C for 10 minutes to thereby extract the yeast cells. The resulting extract was separated from the residue of the yeast cells through centrifugation. In that manner, the extract containing γ-GC was prepared. The extract was concentrated under a reduced pressure of 120 mmHg at a temperature of 50°C, and after kept for 10 hours under the condition, this was concentrated to have a solid concentration of about 30%. The process gave a yeast extraction composition A (yeast extract A). Further, hydrochloric acid was added to the yeast extraction composition A (yeast extract A) to thereby make it have a pH of 4.5, and the resulting concentrated solution was kept at 90°C for about 1 hour thereby giving a cysteine-rich yeast extraction composition B (yeast extract B).

### (Example 6) Evaluation of Yeast Extraction composition (Yeast Extract):

The yeast extraction compositions A and B prepared in Example 5 were separately diluted with hot water at 50°C to give a 1% solution each, and these were organoleptically evaluated. The organoleptic evaluation was made by 10 expert panelists according to a two-point discrimination test method.

**Table 2 Result of Organoleptic Evaluation of Yeast Extraction composition A**

| Test Item | AJ14800 broth extraction composition was stronger. | no difference | AJ14892 broth extraction composition was stronger. |
|---|---|---|---|
| Sulfurous Smell | 0 | 1 | 9 |
| YE Smell | 9 | 1 | 0 |
| YE Taste | 9 | 1 | 0 |

**Table 3 Result of Organoleptic Evaluation of Yeast Extraction composition B**

| Test Item | AJ14800 broth extraction composition was stronger. | no difference | AJ14892 broth extraction composition was stronger. |
|---|---|---|---|
| Sulfurous Smell | 0 | 0 | 10 |
| YE Smell | 9 | 1 | 0 |
| YE Taste | 9 | 1 | 0 |

As a result, it has been known that the strain AJ14892 broth extraction composition has a higher degree of sulfurous smell peculiar to γ-GC (abbreviation of γ-glutamylcysteine) and cysteine, but has a lower degree of YE smell (characteristic yeast smell) and YE taste (characteristic yeast taste) that are the problems to be solved here. Accordingly, it has been known that, by imparting cerulenin resistance to the γ-GC-containing yeast, not only the γ-GC content in the resulting yeast increases but also the resulting yeast extraction composition (yeast extract) has little YE smell and YE taste peculiar to the yeast extract.

The yeast extraction composition B prepared in Example 5 was analyzed, and the amount of the constitutive ingredients per the extract solid fraction was as follows:

**Table 4 Ingredients of AJ14892 Broth Extraction Composition B**

| | |
|---|---|
| Cysteine | 4.10 % |
| Arginine | 0.28 % |
| Lysine | 0.26 % |
| Histidine | 0.00 % |

**Table 5 Ingredients of AJ14800 Broth Extraction Composition B**

| | |
|---|---|
| Cysteine | 2.30 % |
| Arginine | 0.63 % |
| Lysine | 0.61 % |
| Histidine | 0.29 % |

As known from these results, the cysteine content per the extract solid fraction in the strain AJ14892 broth extraction composition B increased as compared with that in the strain AJ14800 broth extraction composition B, while, on the other hand, the basic amino acid (arginine, lysine, histidine) content per the extract solid fraction in the former decreased.

Regarding the tendency, the same results were seen with the strain 10-9, the strain 10-29 and the strain 10-340 randomly selected from the yeasts that had been selected as the yeast strains having resistance to 10 µM or more cerulenin and having an increased γ-GC content in Example 2. In this, the cysteine content in each strain was 3.90%, 3.88% and 3.62%, respectively.

### (Example 7) Composition Change during Washing of Yeast Cells:

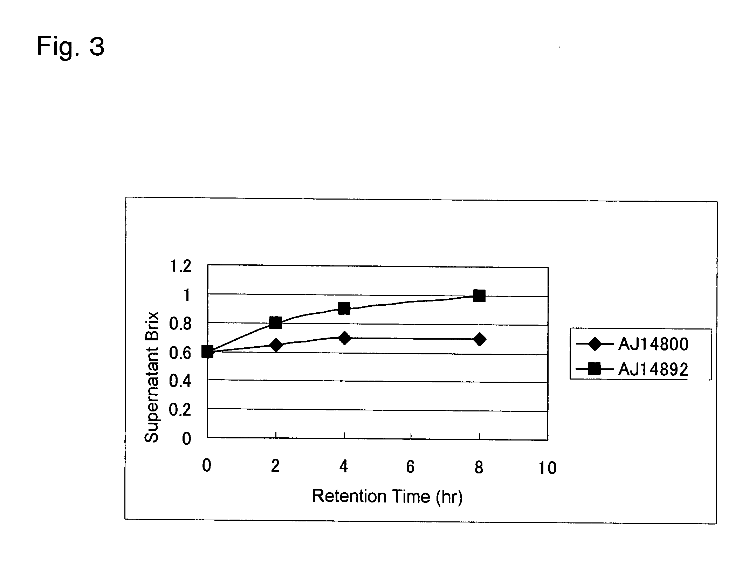

Regarding the AJ14892 broth and the AJ14800 broth, the matter as to whether or not the content of the yeast cells might be removed out of them during washing of the yeast cells was checked in the manner mentioned below. Like in Example 1, the culture broth of the strain AJ14800 described in Example 3 and that of the strain AJ14892 described in Example 4 were centrifuged and washed to thereby prepare a suspension of the light liquid having Brix of 0.6 and having a yeast cell concentration of about 10 g-DCW (dry cell weight)/dl. The suspension was kept at 16°C, with stirring it with a stirrer (at a stirring rate on such a level that the yeast cells would not be settled at the rate). At regular time intervals, the suspension was sampled, centrifuged and the supernatant Brix was measured. Different from that in the strain AJ14800, the supernatant Brix increased with time, though slightly, in the strain AJ14892 (Fig. 3). There was little change in the γ-GC content per the yeast cell weight in the strain AJ14892. Also in this washing step, it may be presumed that the characteristic YE (yeast) compositions could be removed from the cerulenin-resistant yeast cells, and therefore it may be presumed that a yeast cell extraction composition of which the YE taste and the YE smell peculiar to YE were further reduced could be obtained.

### (Reference Example 2) Dissolved Gas in Yeast Cell Broth:

The yeast is facultatively anaerobic, and is generally cultivated with aeration. Accordingly, the culture broth contains air introduced thereinto through aeration and other gases such as carbon dioxide generated by the yeast's respiration dissolved therein. When the yeast cells are extracted, using a CS machine, in such a state where the dissolved gas is in the broth, the dissolved gas may vaporize during extraction, and therefore bubbles may be formed inside the CS machine. As a result, the hot water extraction step with the CS machine may be unstable. To evade the instability, in general, the cells are washed at a temperature higher than the yeast cultivation temperature. As a result, during the cell washing step, the dissolved gas in the broth may be removed and the hot water extraction step may be thereby stabilized.

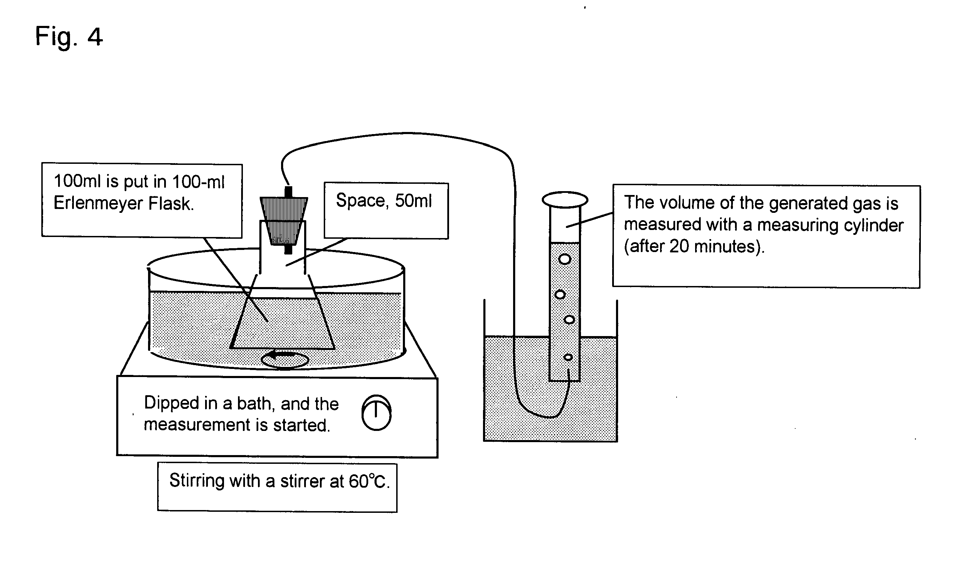

The matter as to how the dissolved gas in the broth would change, depending on the temperature in the cell washing step, was checked as follows:
Like in Example 5, the culture broth of the strain AJ14800 described in Example 3 was centrifuged, and the cells were washed to thereby prepare about 200 ml of a yeast suspension having a supernatant Brix of 0.6 and a yeast cell concentration of about 10 g-DCW/dl. The yeast suspensions, as divided into 100 ml each, were kept at 35°C or 16°C for 4 hours with stirring with a stirrer like in Example 1. The dissolved gas in the suspension was analyzed as follows: 100 ml of the suspension was put into a 100-ml Erlenmeyer flask, and its top was sealed up. In this, the Erlenmeyer flask had a space of about 50 ml. The Erlenmeyer flask was dipped in a water bath at 60°C, and the suspension was held as such for 20 minutes with stirring with a stirrer. The amount of the dissolved gas that had been evaporated during the 20 minutes was measured (Fig. 4).

As a result, the sample held at 35°C for 4 hours contained the dissolved gas in an amount of 29 ml therein, and the sample held at 16°C for 4 hours contained the dissolved gas in an amount of 55 ml therein. From this result, it is known that, when the cells are washed at the temperature 35°C higher than the yeast cultivation temperature, then the dissolved gas in the yeast heavy liquid can be removed, and therefore the amount of the gas to be generated in the hot water extraction step can be reduced and the hot water extraction step can be thereby stabilized. Specifically, the cell washing step at an ordinary temperature of 30°C or higher has the advantage of stabilization of the hot water extraction step.

However, in the invention, a method of washing the cells at a temperature lower than the cultivation temperature, which differs from the ordinary method, is employed whereby the yeast extract improved in point of the smell and the flavor thereof can be obtained, as described in the above Examples.

### (Example 8) Identification of Mutant Gene of Cerulenin-Resistant Strain 10-9:

The strain 10-9 obtained in Example 2 is a mutant selected by utilization of the selection pressure of cerulenin resistance from the natural variants created in the culture of the parent strain AJ14800. To identify the mutant gene, the gene expression cDNA library of the strain 10-9 was formed. The strain 10-9 was cultivated in an SD medium containing 10 µM cerulenin, at 30°C. In the logarithmic growth phase thereof, the cells were collected, and the total-RNA was collected according to an ordinary method. Using the total-RNA and according to the Gateway technique, the full length cDNA expression library was formed. (For the formation of this library, a request was made to Invitrogen Company having the Gateway technique. Using uncut, full-length uncut, and nanoquantity uncut custom cDNA libraries sold by the company (Catalog Nos. 11144-010, 11144-020, and 11649-020), the intended library can be formed.) In this, as the destination vector for the expression library, used was pYES-DEST52 Gateway Vector (Catalog No. 12286-019) by the company. In that manner, a cDNA library containing the gene of the strain 10-9 in the GAL1 promoter was formed.

Next, the cDNA library containing the gene of the strain 10-9 was transformed into a strain S. cerevisiae INVSc1 (by Invitrogen Company, code C81000), according to an ordinary method. For the transformation, used was the competent cell of the strain INVSc1 formed by the use of S.c. EasyComp Transformation Kit (by Invitrogen, code 45-0476), according to the manual booklet. The transformant was inoculated into an SD medium (with a necessary concentration of His, Leu, Trp added thereto) as produced by the use of the same concentration of galactose in place of glucose, and cultivated by shaking at 30°C for 24 hours. All the culture was spread on an SD-agar medium containing 10 µM cerulenin (where galactose was used in place of glucose, and a necessary concentration of His, Leu, Trp was added thereto), and statically cultivated at 30°C for 7 days, whereupon three colonies appeared. The plasmid was collected from these three strains according to an ordinary method. The three plasmids all contained the FAS2 gene of the strain 10-9. In that manner, the FAS2 gene was identified as the gene of the strain 10-9 modified to have cerulenin resistance and have the ability of increasing the γ-GC content in the strain.

### (Example 9) Mutation Analysis of FAS2 Gene:

The base sequence of the FAS2 gene contained in the plasmid obtained according to an ordinary method was determined. There was found a difference from the wild protein of SEQ ID NO: 7 in the Sequence Listing, in that the 1475th glutamic acid in the protein encoded by the FAS2 gene was changed to lysine and the 1800th serine was to asparagine (see SEQ ID NO: 5 and 6 in the Sequence Listing). Therefore, according to an ordinary method, the chromosomal DNA of the strain AJ14800 and that of the strain 10-9 were collected, and the FAS2 gene was amplified and sequenced.

As a result, in both the two proteins encoded by the FAS2 gene of the strain AJ14800 (diploid), the 1475th amino acid was glutamic acid, and the 1800th amino acid was serine. On the other hand, in one protein encoded by the FAS2 gene of the strain 10-9 (diploid), the 1475th amino acid was glutamic acid and the 1800th amino acid was serine; however, in the other protein, the 1475th amino acid was lysine and the 1800th amino acid was asparagine.

### (Example 10) Improvement in Extraction by Two-Stage

### Heating:

The culture broth of the strain AJ14800 described in Example 3 and that of the strain AJ14892 described in Example 4 were extracted according to a method of two-stage heating mentioned below.

The culture broth was separately centrifuged to separate the yeast cells from the culture medium. For the purpose of removing the medium adhering to the cells as the mother liquid, the yeast cells were added to washing water and suspended therein. The resulting suspension was further centrifuged to separate the medium fraction (light liquid) and the yeast cells (heavy liquid). This operation was repeated until Brix of the light liquid reached 0.6. Subsequently, the yeast cells were suspended to have a concentration of about 10 g-DCW (dry cell weight)/dl, and with stirring it with a stirrer (at a stirring rate on such a level that the yeast cells would not be settled at the rate), this was kept at 16°C for 4 hours. Subsequently, the yeast cells were collected through centrifugation. The yeast cells were suspended in water to have a concentration of about 12 g-DCW/dl. The yeast cell suspension was kept heated at a different temperature of from 15 to 70°C for 1 hour for first stage hot extraction. Next, the heating temperature was elevated up to 70 or 80°C, and this was hot extracted for 10 minutes. The extracted yeast cells were centrifuged to separate the supernatant from the extracted cell residue. The supernatant Brix was measured, and the extraction efficiency was compared under different conditions.

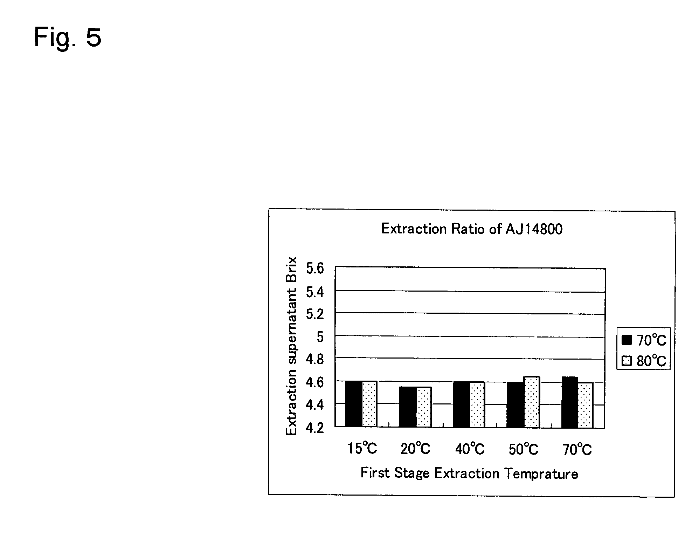

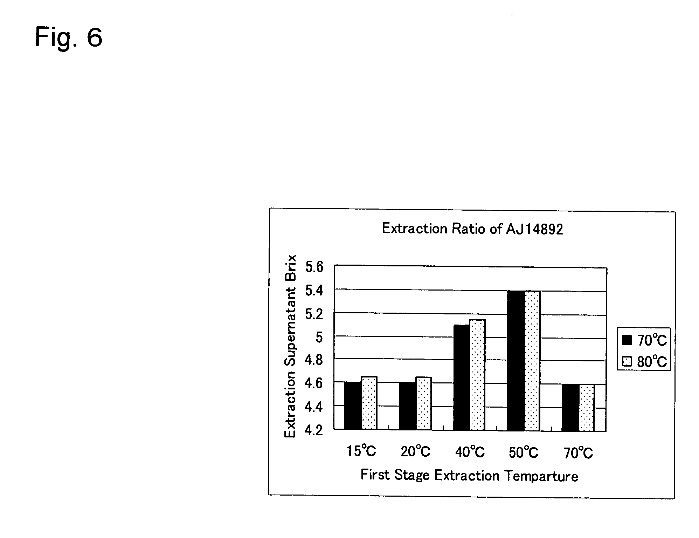

As shown in Fig. 5 and Fig. 6, the extraction ratio did not almost change in the strain AJ14800 not having cerulenin resistance under all the conditions; but in the strain AJ14892 having cerulenin resistance, the extraction ratio increased by the first-stage heating. It has been confirmed that the extraction ratio increased while the first-stage heating time was prolonged up to 14 hours.

### INDUSTRIAL APPLICABILITY

The yeast of the invention having γ-glutamylcysteine accumulated therein, and the yeast extract obtained by the use of the yeast can be widely used for food and drink such as alcohol drinks, bread products, fermented seasonings, etc.

## Claims

1. A yeast having the ability of producing γ-glutamylcysteine and having resistance to cerulenin.

2. The yeast as claimed in claim 1, having resistance to at least 10 µM cerulenin.

3. The yeast as claimed in claim 2, having come to have resistance to at least 10 µM cerulenin as having a mutant FAS2 gene.

4. The yeast as claimed in claim 3, wherein the mutant FAS2 gene is such that the 1475th amino acid in the protein encoded by the natural FAS2 gene of SEQ ID NO: 5 in the Sequence Listing is lysine and/or the 1800th amino acid therein is asparagine.

5. The yeast as claimed in any one of claims 1 to 4, which gained the γ-glutamylcysteine producing ability through modification thereof to lower or lose the intercellular glutathione synthetase activity.

6. The yeast as claimed in any one of claims 1 to 5, which belongs to the genus *Saccharomyces.*

7. The yeast as claimed in claim 6, wherein the yeast is *Saccharomyces cerevisiae*.

8. A method for producing a yeast with γ-glutamylcysteine accumulated therein, by cultivating the yeast of any one of claims 1 to 7 in a culture medium.

9. A method for producing a yeast extract by cultivating the yeast of any one of claims 1 to 7 in a culture medium to thereby accumulate γ-glutamylcysteine in the yeast, and then extracting the yeast.

10. The method for producing a yeast extract as claimed in claim 9, wherein γ-glutamylcysteine is accumulated in the yeast, then the yeast cells are washed at a low temperature, and thereafter the yeast is extracted to give the yeast extract.

11. A method for producing a yeast extract, comprising further processing the yeast extract of claim 9 or 10 for heat treatment or enzymatic treatment to thereby change a part or all of γ-glutamylcysteine in the yeast extract into cysteine.

12. The method for producing a yeast extract of as claimed in claim 11, wherein the cysteine content per the yeast extract on the dry matter base is at least 3.5% and the basic amino acid content is at most 1.0%.

13. A food or drink produced by mixing the yeast extract of any one of claims 9 to 12 in a raw material of food or drink, and then processing it.

14. The method for producing a yeast extract as claimed in claim 9, wherein the step of accumulating γ-glutamylcysteine in a yeast, then washing the yeast cells at a low temperature and extracting them to give an yeast extract includes a step of heating the yeast cells in two stages each having a different temperature range to give the extract.

15. The method for producing a yeast extract as claimed in claim 14, wherein in the step of heating the yeast cells in two stages each having a different temperature range to give the extract, the first-stage extraction temperature is from 25 to 60°C and the second-stage extraction temperature is from 65 to 99°C.
